# EUROPEAN PATENT APPLICATION

(11) **EP 2 568 403 A1**
(43) Date of publication of application: **13.03.2013**
(21) Application number: 11425229.9
(22) Date of filing: 09.09.2011
(51) Int. Cl.: G06F 19/00

(54) **Method and system for managing data related to an individual wearing a protection helmet**

(71) Applicant: Trizero S.r.l., 23851 Galbiate (LC) (IT)
(72) Inventor: Polvara, Fabio, 23851 Galbiate (Lecco) (IT)
(74) Representative: Baroni, Matteo

(57) **Abstract**

A method of managing data in electronic format related to an individual wearing a protection helmet, comprising: affixing a code (10) on a surface of a protection helmet (11) of an individual; providing a first device (100) equipped with: a reading module (110) for reading and interpreting said code (10), outputting corresponding decoded information (DI); a data entry module (120) to enable a user to input main data (MD) comprising one or more pieces of information related to said individual, said pieces of information comprising at least one from: personal data, blood group, allergies, past illnesses or pathologies still in progress, contacts in case of need; a transmission module (130) for generating a main signal (MS) incorporating said decoded information (DI) and main data (MD); providing a storage apparatus (200), comprising: a reception module (210) for receiving said main signal (MS); a memory (220) for storing said decoded information (DI) and main data (MD) associated with each other; providing a second device (300) equipped with: a reading module (310) for reading and interpreting the code (10) affixed on said helmet (11), thus obtaining said decoded information (DI); a transmission module (320) for sending a request signal (RS) incorporating said decoded information (DI) to said storage apparatus (200). The storage apparatus (200) is configured for: receiving said request signal (RS); identifying in said memory (220), the main data (MD) associated with said decoded information (DI); sending an answer signal (AS) incorporating said main data (MD) to said second device (300).

## Description

The present invention relates to a method of managing data in electronic format related to an individual wearing a protection helmet.

The invention also relates to a system for managing data in electronic format related to an individual wearing a protection helmet.

It is known that when an accident occurs in which an individual is implicated, it is a fundamental requirement to be able to collect all information necessary to give the appropriate help to that individual.

This information can comprise not only contacts with relatives or individuals that in any case are close to the wounded or injured person, but also data relating to the blood group, possible allergies, possible past illnesses or pathologies still in progress, etc.

Presently, these pieces of information generally cannot be found in a quick and reliable manner; the relatives' references and contacts can be present on the individual's mobile phone needing help but they cannot be always easily identified; data relating to the blood group can be present on a metal plate worn on the individual's neck, but this habit does not belong to all people; it is substantially impossible to immediately know details relating to allergies and pathologies.

Clearly, this can have very serious consequences, since, in the absence of these data, it is impossible to offer an effective first aid and, on the contrary, the absence of information about allergies and pathologies can make some interventions on the spot even dangerous.

It is an aim of the present invention to provide a method and a system enabling the first aid operations performed on an injured individual to be really effective and above all not dangerous.

The above and further aims are substantially achieved by the method and system according to that which is disclosed in the appended claims.

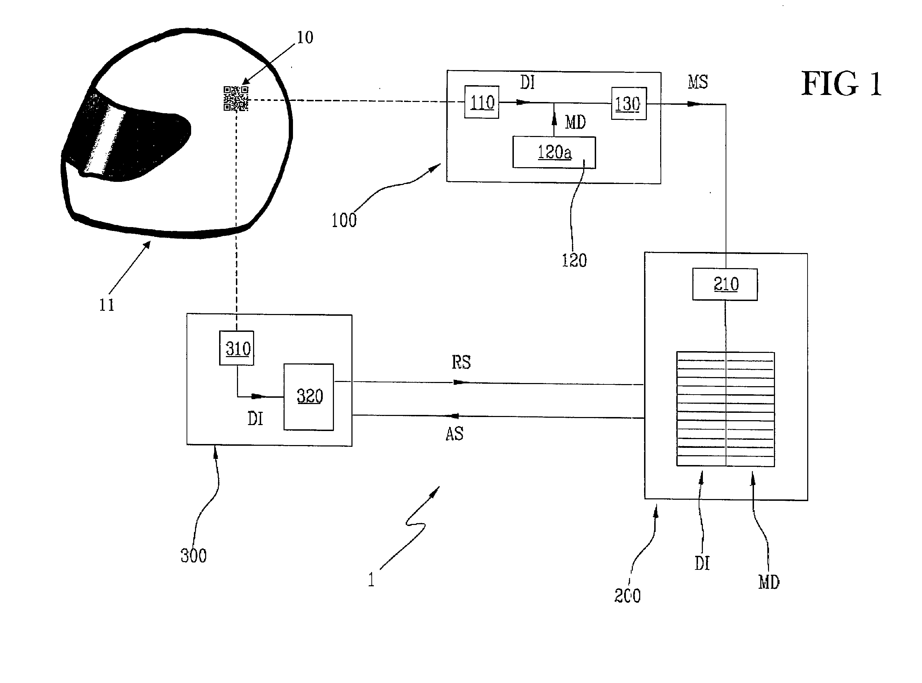

Further features and advantages will become more apparent from the description of a preferred but not exclusive embodiment of the invention. This description is provided hereinafter with reference to the accompanying figure 1, also given by way of nonlimiting example, in which a block diagram of the system according to the invention is shown.

With reference to the accompanying figure 1, a system in accordance with the present invention has been generally denoted at 1.

As said, the method put into practice by system 1 is the object of the present invention as well.

System 1 first of all comprises a protection helmet 11. By way of example only, helmet 11 can be a crash helmet for motorcyclists, motorists, cyclists, canoeists, members of the fire brigade, individuals operating in building yards or areas where wearing a protection helmet is compulsory/appropriate, etc. Generally, the protection helmet 11 can/must be worn by an individual in places and circumstances where there is a risk of injury to the head of the individual himself/herself.

On such a helmet 11 a respective code 10 is affixed. Preferably, code 10 is printed on adhesive material consisting of an adhesive label, for example. Preferably, code 10 is a QR code. The specifications relating to this types of codes are known by themselves and illustrated in the web site www.qrcode.com, for example. Practically, they are codes that are graphically able to represent an unequivocal coding of a given alphanumeric string. Within the scope of the present invention code 10 is preferably generated starting from an alphanumeric string generated randomly, or according to a predetermined sequence. Practically, code 10 employed in the present invention is directly representative of contents known by themselves substantially devoid of meaning at the moment they are generated.

As better clarified in the following, the information associated with code 10 will be defined by the system users.

System 1 further comprises a first device 100, preferably of the electronic type.

The first device 100 comprises a reading module 110 for reading and interpreting the aforesaid code 10.

Preferably, the reading module 110 can comprise a detecting device, such as a camera for example, and a processing unit, suitably programmed for recognising code 10 and output the corresponding decoded information DI therefrom.

In other words, the reading module 110, when activated in correspondence with code 10, is able to give back the decoded corresponding information DI, i.e. the starting alphanumeric string for generation of code 10 in graphic form.

Referring particularly to codes QR, the algorithm underlying generation and interpretation of the codes is known by itself and therefore will not be described herein in detail.

The first device 100 further comprises a data entry module 120, enabling a user to input main data MD. In a preferred embodiment, the main data MD are voice data.

Practically, the data entry module 120 enables the user to input data through speaking; that which is said by the user in this situation can be received, stored and processed by the data entry module 120.

By way of example, the data entry module 120 can comprise a converter 120a to convert the voice signal inputted by the user to an electronic format, thus obtaining said main data MD.

In addition or alternatively, the main data MD can comprise written data inputted by the user through a keyboard, for example.

According to the invention, the main data MD comprise one or more pieces of information associated with a person, i.e. the owner of helmet 11 or the individual using it. These pieces of information comprise at least one from: personal data, blood group, allergies, past illnesses or pathologies still in progress, contacts in case of need.

The personal data may comprise one or more of the following information: name, surname, place/year of birth, place of residence (commune and province), identity card number.

Contacts in case of need may comprise contacts relating to a first person to be contacted and optionally contacts relating to a second person to be contacted, if the first person is not available/cannot be found.

The first device 100 further comprises a transmission module 130, configured for generating a main signal MS. The main signal MS incorporates the decoded information DI, supplied by the reading module 110 and the main data MD inputted by the user.

The main signal MS is sent to a storage apparatus 200 to be described in the following.

Preferably, connection between the transmission module 130 and storage apparatus 200 is of the remote type. Preferably, connection between the transmission module 130 and storage apparatus 200 is of the wireless type.

The first device 100 in the preferred embodiment can consist of, or comprise a smartphone, i.e. a mobile phone of new generation. This device can advantageously employ its own camera integrated thereinto to detect code 10; a suitable application, loaded in the device processor, carries out interpretation of the code and achievement of the corresponding alphanumeric string.

By a microphone (necessarily present in a telephone set) the user can input the main data MD that can then be suitably processed by said processor.

Then the main signal MS is generated; this signal through the available connectivity, preferably through the Internet, is sent to apparatus 200 so that apparatus 200 can receive the alphanumeric string (i.e. the decoded information DI) and the main data MD. In this specific context, the transmission module 130 can comprise all circuits/devices present within the smartphone that help in carrying out connection between the first device 100 and the storage apparatus 200.

The storage apparatus 200 first of all comprises a reception module 210 for receiving the main signal MS. Practically, the reception module 210 allows a connection, preferably of the wireless and remote type, with the first device 100 and in particular with the transmission module 130 being part of the first device 100.

By way of example, the transmission module 130 and/or reception module 210 can use the GSM technology, UMTS technology, etc.

The storage apparatus 200 further comprises a memory 220 for storing the decoded information DI and the main data MD associated with each other, i.e. the assembly of data received by means of the main signal MS.

In the preferred embodiment, the storage apparatus 200 is configured for enabling data relating to a plurality of individuals to be stored. Therefore, from a logical point of view, memory 220 can be structured like a table in which each row is dedicated to a respective code 10; in particular, each row contains the alphanumeric string representative of a given code (i.e. the above mentioned decoded information DI), together with the main data MD that have been transmitted together with such a string.

In this manner, the memory 220 of the storage apparatus 200 maintains the association between each code and the pieces of information that have been associated therewith.

The storage apparatus 200 conveniently comprises a processing unit (not shown) suitably programmed for managing the reception module 210 and memory 220 according to that which is described above. This processing unit further enables accomplishment of the operations for an answer to a second device to be described in the following.

The storage apparatus 200 practically can be made as an electronic computer, like a server for example, or an assembly of electronic computers programmed for carrying out the herein described and hereinafter claimed operations.

Advantageously, system 1 further comprises a second device 300, preferably of the electronic type. The second devise 300 in turn can comprise a reading module 310 for reading and interpreting the aforesaid code 10. Preferably, the reading module 310 can comprise a detecting device such as a camera, and a processing unit suitably programmed for recognising code 10 and retrieving the corresponding decoded information DI. In other words, the reading module 310, when activated in correspondence with code 10, is able to return the corresponding decoded information DI, i.e. the starting alphanumeric string for generation of code 10 in a graphic form.

Preferably, the reading module 310 of the second device 300 has the same structure and operation as the reading module 110 of the first device 100.

The second device 300 further comprises a transmission module 320 for sending the storage apparatus 200 a request signal S incorporating the decoded information DI. Preferably, connection between the second device 300, and in particular the transmission module 320, and the storage apparatus 200 is of the remote type.

Preferably, connection between the second device 300, and in particular the transmission module 320, and the storage apparatus 200 is of the wireless type.

The storage apparatus 200, on receipt of the request signal RS, identifies in memory 220, the main data MD associated with the decoded information DI contained in the request signal RS. Practically, apparatus 200 in memory 220 identifies the row in which the decoded information DI is and identifies the main data MD that are on the same row.

Subsequently, apparatus 200 will send an answer signal AS incorporating the identified main data MD to the second device 300. Preferably, transmission of the answer signal AS is carried out through a connection of the remote type.

Preferably, transmission of the answer signal AS is carried out through a connection of the wireless type.

From the point of view of operation, the following is to be pointed out.

The first device 100 is positioned in the vicinity of code 10 present on the surface of helmet 11. Code 10 is therefore read and interpreted by the first device 100 so as to go back to the decoded information DI.

The user will then input the main data MD that, as said, can comprise at least one of the following pieces of information: personal data, blood group, allergies, past illnesses or pathologies still in progress, contacts in case of need.

Through the first device 100 therefore an association between code 10 and the main data MD inputted by the user is created.

Through the aforesaid main signal MS the decoded information DI and main data MD are sent to the storage apparatus 200. The storage apparatus 200 keeps this information stored.

Should it be necessary to retrieve the main data MD, i.e. should it be necessary to give help to the injured individual wearing helmet 11, and should not said injured individual be conscious, the second device 300 can be used. The second device 300 in fact is placed close to helmet 11, and in particular close to code 10. The latter is read and interpreted, obtaining the decoded information DI.

Through the request signal RS, the decoded information DI is sent to the storage apparatus 200 that, in reply, supplies said answer signal AS incorporating the main data MD associated with the decoded information DI incorporated into the request signal.

Still by means of the second device 300 it is finally possible to take advantage of the received main data MD and thus the injured individual can receive help taking into account all important details for his/her health.

By virtue of the fact that the concerned data (i.e. the decoded information DI and main data MD) are stored in the storage apparatus 200, distinct and physically separated from the first and second devices 100, 300, storage and retrieval of these data can also be carried out through devices different from each other (provided they have the minimum features for performing the herein described operations), and without important restrictions to the geographical position where the user is.

For instance, the first device 100 can be owned by the motorcyclist who inputs his/her data in the system, in the form of main data MD. On the contrary, the second device 300 can be used by a rescuer, in the event that unfortunately the injured individual would need assistance.

It is to be noted that the first device 100 and second device 300 have been shown separately only for the sake of clarity and simplicity in discussing the matter. Practically, the first and second devices 100, 300 can be incorporated into the same apparatus (a smartphone, for example) by which first the data associated with a given code are inputted and, subsequently, the stored data are retrieved so as to be able to take advantage of them. In this context, it is possible to say that the first device 100 and second device 300 represent two distinct operating modes of a same apparatus. Advantageously, it is provided that this apparatus be equipped with a single reading module that can be used both in the first operating mode (data input), and in the second operating mode (retrieval of the previously inputted data).

Likewise, a single transceiver structure can be employed for connection with the storage apparatus 200, in the first and second operating modes.

It should be also recognised that the first and second devices 100, 300 can also be physically incorporated into two distinct apparatuses.

For instance, each of the two devices 100, 300 can consist of a smartphone. In this case, the information inputted by a user through the first device 100 can be retrieved by another user through the second device 300, like in the above mentioned case relating to the injured person and the rescuer.

The invention achieves important advantages.

First of all the method and system according to the invention enable quick and reliable retrieval of the essential data relating to an individual, so as to allow first aid operations to be carried out which are really effective and above all not dangerous.

In addition, the invention can be put into practice through means that presently can be easily found (more precisely, widespread means such as the smartphones) and with very reduced prices.

## Claims

1. A method of managing data in electronic format related to an individual, comprising:
- affixing a code (10) on a surface of a protection helmet (11) associated with an individual;
- providing a first device (100) equipped with:
• a reading module (110) for reading and interpreting said code (10), outputting corresponding decoded information (DI);
• a data entry module (120) to enable a user to input main data (MD) comprising one or more pieces of information associated with said individual, said pieces of information comprising at least one from: personal data, blood group, allergies, past illnesses or pathologies still in progress, contacts in case of need;
• a transmission module (130) for generating a main signal (MS) incorporating said decoded information (DI) and main data (MD);
- providing a storage apparatus (200), comprising:
• a reception module (210) for receiving said main signal (MS);
- a memory (220) for storing said decoded information (DI) and main data (MD) associated with each other;
- providing a second device (300) equipped with:
• a reading module (310) for reading and interpreting the code (10) affixed on said helmet (11), thus obtaining said decoded information (DI)
• a transmission module (320) for sending a request signal (RS) incorporating said decoded information (DI) to said storage apparatus (200);
said storage apparatus (200) being configured for:
• receiving said request signal (RS);
• identifying in said memory (220), the main data (MD) associated with said decoded information (DI);
• sending an answer signal (AS) incorporating said main data (MD) to said second device (300).

2. A method as claimed in claim 1, wherein said code (10) is a QR code.

3. A method as claimed in claim 1 or 2, wherein said data entry module (120) comprises a converter (120a) to convert a voice signal inputted by said user into said main data (MD) in electronic format.

4. A method as claimed in anyone of the preceding claims, further comprising:
- generating a random or progressive alphanumeric string;
- generating said code (10) as coded graphic representation of said alphanumeric string, said alphanumeric string defining said decoded information (DI).

5. A method as claimed in claim 4, wherein the step of generating said code (10) is performed before activation of said first device (100) for inputting said main data (16).

6. A system for managing data in electronic format related to an individual, comprising:
- a protection helmet (1), a code (10) being affixed on a surface of said helmet (11);
- a first device (100) equipped with:
• a reading module (110) for reading and interpreting said code (10), outputting corresponding decoded information (DI);
• a data entry module (120) to enable a user to input main data (MD) comprising one or more pieces of information associated with said individual, said pieces of information comprising at least one from: personal data, blood group, allergies, past illnesses or pathologies still in progress, contacts in case of need;
• a transmission module (130) for generating a main signal (MS) incorporating said decoded information (DI) and main data (MD);
- a storage apparatus (200), comprising:
• a reception module (210) for receiving said main signal (MS);
- a memory (220) for storing said decoded information (DI) and main data (MD) associated with each other;
- a second device (300) equipped with:
• a reading module (310) for reading and interpreting the code (10) affixed on said helmet (11), thus obtaining said decoded information (DI)
• a transmission module (320) for sending a request signal (RS) incorporating said decoded information (DI) to said storage apparatus (200);
said storage apparatus (200) being configured for:
• receiving said request signal (RS);
• identifying in said memory (220), the main data (MD) associated with said decoded information (DI);
• sending an answer signal (AS) incorporating said main data (MD) to said second device (300).

7. A system as claimed in claim 6, wherein said codes (10) are QR codes.

8. A system as claimed in claim 6 or 7, wherein said data entry module (120) comprises a converter (120a) to convert a voice signal inputted by said user into said main data (MD) in electronic format.

9. A system as claimed in anyone of claims 6 to 8, wherein said first device (100) and second device (300) are integrated into a single apparatus.

10. A system as claimed in anyone of claims 6 to 8, wherein said first device (100) and second device (300) are integrated into apparatuses that are distinct from each other.

11. A system as claimed in anyone of claims 6 to 10, wherein each of said codes (10) is a coded graphic representation of a random or progressive alphanumeric string, the latter defining said decoded information (DI).

12. An electronic device (100), comprising:
• a reading module (110) for reading and interpreting a code (10), affixed on a protection helmet (11) associated with an individual, outputting corresponding decoded information (DI);
• a data entry module (120) to enable a user to input main data (MD) comprising one or more pieces of information associated with said individual, said pieces of information comprising at least one from: personal data, blood group, allergies, past illnesses or pathologies still in progress, contacts in case of need;
• a transmission module (130) for generating a main signal (MS) incorporating said decoded information (DI) and main data (MD), said main signal (MS) being sent to a storage apparatus (200) configured for receiving said main signal (MS) and storing said decoded information (DI) and main data (MD) associated with each other.

13. A storage apparatus (200), comprising:
• a reception module (210) for receiving a main signal (MS) sent by a first device (100) and incorporating a decoded information (DI), obtained from reading and interpretation of a code (10) affixed on a protection helmet (11) associated with an individual, and main data (MD) comprising one or more pieces of information related to said individual, said pieces of information comprising at least one from: personal data, blood group, allergies, past illnesses or pathologies still in progress, contacts in case of need;
• a memory (220) for storing said decoded information (DI) and main data (MD) associated with each other; said storage apparatus (200) being configured for:
• receiving from a second device (300), a request signal (RS) incorporating the decoded information (DI) obtained from reading and interpretation of said code (10);
• identifying in said memory (220), the main data (MD) associated with said decoded information (DI);
• sending an answer signal (AS) incorporating said main data (MD) to said second device (300).

14. An electronic device (300) equipped with:
• a reading module (310) for reading and interpreting a code (10) affixed on said protection helmet (11) associated with an individual, thus obtaining the respective decoded information (DI)
• a transmission module (320) for sending a request signal (RS) incorporating said decoded information (DI) to a storage apparatus (200);
said device (300) being configured for receiving from said storage apparatus (200), an answer signal (AS) incorporating main data (MD comprising one or more pieces of information related to said individual, said pieces of information comprising at least one from:
personal data, blood group, allergies, past illnesses or pathologies still in progress, contacts in case of need;
said main data (MD) being identified by said apparatus (200) in a memory (220) thereof containing the decoded information (DI) incorporated in said request signal (RS) and said main data (MD), associated with each other.
